(19) Europäisches Patentamt
European Patent Office
Office européen des brevets

(11) Veröffentlichungsnummer : **0 093 443**
**B1**

# (12) EUROPÄISCHE PATENTSCHRIFT

(45) Veröffentlichungstag der Patentschrift : **03.04.85**

(51) Int. Cl.⁴ : **C 07 C119/20**

(21) Anmeldenummer : **83104264.3**

(22) Anmeldetag : **30.04.83**

## (54) 4-Halomethylbenzoesäure-alkylester-imine und Verfahren zu deren Herstellung.

(30) Priorität : **05.05.82 DE 3216722**

(43) Veröffentlichungstag der Anmeldung : **09.11.83 Patentblatt 83/45**

(45) Bekanntmachung des Hinweises auf die Patenterteilung : **03.04.85 Patentblatt 85/14**

(84) Benannte Vertragsstaaten : **BE CH DE FR GB IT LI NL**

(56) Entgegenhaltungen :
**FR-A- 2 323 378**
**US-A- 2 877 269**
**Chemical Abstracts Band 66, Nr. 3, 16. Januar 1967, Columbus, Ohio, USA B.G. BACCAR et al. "Structure and reactions of some substituted hydrazidines", Seite 1019, Spalte 2, Abstract Nr. 10677d In Verbindung mit Chemical Abstracts 8th Collective Index: subjects temp-tripep, Seite 31646r**

(73) Patentinhaber : **HOECHST AKTIENGESELLSCHAFT**
**Postfach 80 03 20**
**D-6230 Frankfurt am Main 80 (DE)**

(72) Erfinder : **Heiss, Lorenz, Dr.**
**Stormstrasse 39**
**D-6238 Hofheim am Taunus (DE)**

**EP 0 093 443 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Gegenstand der Erfindung sind neue (4-Halomethyl)-imino-benzoesäurealkylester der Formel

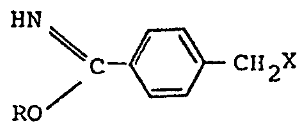

und deren HCl oder HBr-Salze, wobei R $C_1$-$C_4$-Alkyl oder $C_1$-$C_4$-Alkoxyäthyl und X Cl oder Br bedeutet.

Diese Verbindungen werden hergestellt durch Reaktion von p-Cyanbenzylchlorid oder p-Cyanbenzylbromid mit einem $C_1$-$C_4$-Alkohol oder $C_1$-$C_4$-Alkoxyäthanol in Gegenwart von Chlor- oder Bromwasserstoff. Die Reaktion wird so durchgeführt, daß man 1 Mol p-Cyanbenzylhalogenid in einem Überschuß von ca. 1,5 bis 5 Mol des Alkohols dispergiert und während ca. 10 bis 30, vorzugsweise ca. 20 Stunden bei einer Temperatur von 0 bis 30, vorzugsweise 5 bis 20 °C in dieses Reaktionsgemisch Chlorwasserstoff oder Bromwasserstoff einleitet. Die Menge an Halogenwasserstoff ist unkritisch, mindestens jedoch die äquivalente Menge, optimal ist aber eine Menge von 2 Mol Halogenwasserstoff auf ein Mol Cyanbenzylhalogenid. Nach Beendigung der Reaktion wird der überschüssige Halogenwasserstoff entfernt oder neutralisiert und der Überschuß an Alkohol abdestilliert. Man erhält dann die (4-Halogenmethyl)-imino-benzoesäurealkylester in Form ihrer HCl oder HBr-Salze. Diese Salze können direkt weiterverarbeitet werden. Will man die freien Alkylester-imine erhalten, muß man die Salze nach bekannten Methoden noch mit einer äquivalenten Menge einer Base umsetzen.

Durch Umsetzung mit o-Aminophenolen, o-Aminothiophenolen oder o-Phenylendiaminen lassen sich aus diesen (4-Halomethyl)-imino-benzoesäure-alkylester bzw. deren Salzen 2-Benzoxazolyl-4-halomethylbenzole und deren Thiazolyl- und Imidazolyl-Analoga herstellen. Diese letztgenannten Verbindungen sind wertvolle Ausgangsverbindungen für die Herstellung von optischen Aufhellern aus der Reihe der 4,4'-Di-oxazolyl-2-stilbene (Khim. Geterotrikl. Soedin Bd. 1981, 463-467) sowie für die Herstellung von durch UV-Strahlung polymerisierten Massen (US-PS 3 912 606) und für UV-Stabilisatoren (US-PS 4 075 162).

Beispiel 1

75,8 g (0,5 Mol) p-Cyanbenzylchlorid werden in 32 g (1 Mol) Methanol dispergiert und bei 5-15 °C Ca. 35,5 g (1 Mol) Chlorwasserstoff eingeleitet. Man rührt ca. 20 Stunden nach bis im Infrarot-spektrum die Nitrilbande bei 2 220 $cm^{-1}$ verschwunden ist. Nach Entfernung des Methanols und Chlorwasserstoffs erhält man 110 g (0,5 M) 4-Chlormethyl-benzimidomethyläther-hydrochlorid.

Ausbeute : quantitativ

| | | | | |
|---|---|---|---|---|
| gefunden : | C 49,0 % | H 5,0 % | Cl 32,0 % | $Cl^-$ 16,1 % |
| berechnet : | C 49,2 % | H 5,0 % | Cl 32,2 % | $Cl^-$ 16,1 % |

Beispiel 2

98 g (0,5 Mol) p-Cyanbenzylbromid werden in 46 g (1 Mol) Äthanol dispergiert und bei 20 bis 25 °C ca. 32 g (0,9 Mol) Chlorwasserstoff eingeleitet. Man rührt ca. 18 Stunden nach bis im IR-Spektrum die Nitrilbande bei 2 220 $cm^{-1}$ verschwunden ist. Nach Entfernung des Äthanols und Chlorwasserstoffs erhält man 139 g (0,5 Mol) 4-Brommethylbenzimidoäthylmäther-hydrochlorid.

Ausbeute : quantitativ.

| | | | | |
|---|---|---|---|---|
| gefunden : | C 43,0 % | H 4,8 % | Br 28,2 % | $Cl^-$ 12,5 % |
| berechnet : | C 43,1 % | H 4,7 % | Br 28,7 % | $Cl^-$ 12,7 % |

**Ansprüche**

1. (4-Halomethyl)-imino-benzoesäure-alkylester der Formel

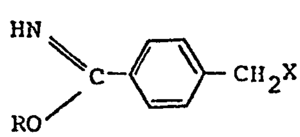

und deren HCl oder HBr-Salze, wobei R $C_1$-$C_4$-Alkyl oder $C_1$-$C_4$-Alkoxyäthyl und X Cl oder Br bedeutet.

2. Verfahren zur Herstellung von 4-Halomethylbenzoesäurealkylester-iminen nach Anspruch 1,

dadurch gekennzeichnet, daß man p-Cyanbenzylchlorid oder p-Cyanbenzylbromid mit einem $C_1$-$C_4$-Alkohol oder $C_1$-$C_4$-Alkoxyäthanol in Gegenwart von Chlor- oder Bromwasserstoff umsetzt und gegebenenfalls aus den so erhaltenen Chlorid- oder Bromid-Salzen das freie Imin mit einer Base freisetzt.

## Claims

1. An alkyl (4-halomethyl) iminobenzoate of the formula

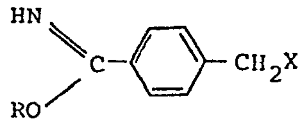

and its HCl or HBr salts, R denoting $C_1$-$C_4$-alkyl or $C_1$-$C_4$-alkoxyethyl and X denoting Cl or Br.

2. A process for the preparation of an imine of an alkyl 4-halomethylbenzoate as claimed in claim 1, which comprises reacting p-cyanobenzyl chloride or p-cyanobenzyl bromide with a $C_1$-$C_4$-alcohol or $C_1$-$C_4$-alkoxyethanol in the presence of hydrogen chloride or bromide and optionally liberating the free imine with a base from the chloride or bromide salts thus obtained.

## Revendications

1. Halogéno-méthyl-4 benzène-carboximidates d'alkyles qui répondent à la formule :

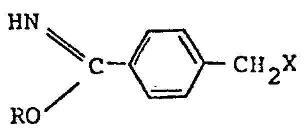

dans laquelle R représente un radical alkyle contenant de 1 à 4 atomes de carbone ou un radical alcoxy-éthyle dont la partie alcoxy contient de 1 à 4 atomes de carbone et X représente Cl ou Br, ainsi que les sels qu'ils forment avec HCl ou HBr.

2. Procédé de préparation d'halogéno-méthyl-4 benzène-carboximidates d'alkyles selon la revendication 1, procédé caractérisé en ce qu'on fait réagir le chlorure de cyano-4 benzyle ou le bromure de cyano-4 benzyle avec un alcool en $C_1$-$C_4$ ou un ($C_1$-$C_4$) alcoxy-éthanol, en présence de chlorure ou de bromure d'hydrogène, et, à partir des chlorhydrates ou bromhydrates ainsi obtenus, on libère éventuellement l'imine libre au moyen d'une base.